Europäisches Patentamt

**European Patent Office**

**Office européen des brevets**

(11) Veröffentlichungsnummer: **0 021 123**
**B1**

(12) **EUROPÄISCHE PATENTSCHRIFT**

(45) Veröffentlichungstag der Patentschrift:
**20.07.83**

(51) Int. Cl.³: **A 61 M 11/00, B 05 B 11/02**

(21) Anmeldenummer: **80103035.4**

(22) Anmeldetag: **31.05.80**

(54) **Sprühapplikator.**

(30) Priorität: **08.06.79 DE 7916475 U**

(43) Veröffentlichungstag der Anmeldung:
**07.01.81 Patentblatt 81/1**

(45) Bekanntmachung des Hinweises auf die Patenterteilung:
**20.07.83 Patentblatt 83/29**

(84) Benannte Vertragsstaaten:
**AT BE CH DE FR GB IT LI LU NL SE**

(56) Entgegenhaltungen:
**DE-A-2 028 314**
**DE-A-2 157 582**
**DE-A-2 349 940**
**DE-A-2 709 796**
**DE-A-2 802 771**
**DE-B-2 012 607**
**DE-U-7 916 475**
**FR-A-2 267 798**
**US-A-3 467 097**
**US-A-4 067 499**

(73) Patentinhaber: **HOECHST AKTIENGESELLSCHAFT,**
**Postfach 80 03 20, D-6230 Frankfurt/Main 80 (DE)**

(72) Erfinder: **De Felice, Wilfried, Im Stückes 26,**
**D-6233 Kelkheim (Taunus) (DE)**

ACTORUM AG

## Sprühapplikator

Gegenstand der Erfindung ist ein Sprühapplikator für flüssige oder pulverförmige Arzneimittel zur einmaligen Anwendung, der eine genaue Dosierung des Arzneimittels in den Nasalbereich oder an anderen Körperstellen erlaubt.

Vorrichtungen zum Versprühen einer Flüssigkeit aus einem geschlossenen Behältnis miteiner handbetätigten Saug-/Druck-Pumpe und einer Sprühdüse sind in vielen Ausführungsformen bekannt. So ist in der deutschen Offenlegungsschrift 2 709 796 ein Sprühapplikator, bestehend aus einem geschlossenen Behältnis mit einer handbetätigten Saug-/Druck-Pumpe, einem Sprühkopf mit einer sich koaxial erstreckenden Ausnehmung und einer seitlich angeordneten Düse sowie einem die Pumpenkammer abschliessenden Überdruckventil, bei dem als Verschlussstück ein Ventilschieber verwendet wird, beschrieben.

Sprühapplikatoren dieser und ähnlicher Bauart weisen einen komplizierten technischen Aufbau auf und zeigen bei Kleinstdosierungen in der Grössenordnung von etwa 0,1 ml extrem hohe Dosierschwankungen pro Sprühstoss. Häufig wird auch bei derartigen Sprühapplikatoren beim Betätigen des Pumpenhubes ein sogeanntens «Stottern» beobachtet, was sich im Sprühbild durch eine unregelmässige Tröpfchengrösse bemerkbar macht. Hinzu kommt, dass bekannte Sprühapplikatoren die eigentliche Dosierkammer oft schlecht abdichten, so dass bereits nach wenigen Tagen Lagerung der Inhalt zum Teil verdunstet ist. Eine genaue Dosierung des Arzneimittels ist dann natürlich nicht mehr möglich.

Hinzu kommt bei einer ungenügenden Abdichtung die Gefahr, dass in die oft sterilen Zubereitungen Mikroorganismen einwandern können, wodurch die Sterilität verlorengeht und Zersetzungen des Arzneimittels eintreten können.

Aus der DE-A-2 349 940 ist ein Sprühgerät bekannt, bei dem auf den Nadelkonus einer Injektionsspritze ein Sprühdüsenansatz aufgesteckt wird. Dieser besteht aus einem Düsenkörper mit einer durchgehenden Bohrung, in deren vorderes Ende eine Düse mit einer kleinen zentralen Öffnung eingesetzt ist. Bis nahe an diese Öffnung erstreckt sich ein parallel zur Längsachse des Düsenkörpers gehalterter Stift, der an seinem rückwärtigen Ende durch Stege mit dem Düsenkörper verbunden ist. Solche Vorrichtungen sind kompliziert im Aufbau und damit aufwendig in der Herstellung. Ausserdem erlauben sie nicht eine wirksame Versprühung kleinster Mengen. Darüber hinaus erfolgt die Dosierung des zu versprühenden Gutes mit der Injektionsspritze, was eine Handhabung durch geschultes Personel voraussetzt.

Es stellte sich deshalb die Aufgabe, einen diese Nachteile überwindenden Sprühapplikator zu entwickeln. Es wurde nun gefunden, dass ein Sprühapplikator für flüssige oder pulverförmige Arzneimittel zur einmaligen Anwendung, bei dem der das Arzneimittel enthaltende Behälter (1) an der einen Seite mit einem Kolbenstopfen (2) und auf der anderen Seite mit einem Sprühaufsatz (3) versehen ist, der eine von einer Sprühdüse (7) gebildete Wirbelkammer (8) aufweist, besonders vorteilhaft ist, wenn der Behälter (1) aus einem oder mehreren Dosierbereich(en) besteht, und der Sprühaufsatz mit einer Verschlusskappe (4) versehen ist, durch einen umlaufenden Dichtwulst (5) gegenüber dem Behälterrand abdichtend wirkt und einen rechtwinkligen Austrittskanal (6) aufweist, durch den der gesamte Arzneimittelstrom in eine radiale Richtung abgelenkt wird, und durch den der Arzneimittelstrom rechtwinklig in die Wirbelkammer (8) geleitet und von dort nach aussen gesprüht wird.

In den Kolbenstopfen wird eine Kolbenstange (12) eingeschraubt oder eingeschnappt. An der Spitze des Sprühaufsatzes befindet sich mittig die Sprühdüse, die in der Aufnahmebohrung befestigt ist. Die Verschlusskappe mit dem im Innern angespritzten umlaufenden Dichtkonus (13) dient zur Abdichtung des Behältnisses und verhindert den vorzeitigen Austritt des Arzneimittels. Die Verschlusskappe erhält ihren festen Sitz durch Nokken (14), die beim Audrücken des Verschlusses in eine am Sprühaufsatz angebrachte Nut (15) einrasten.

Weiterhin ist die Verschlusskappe am unteren Rand mit einem umlaufenden erhabenen Ring (16) versehen, um das Abziehen der Verschlussskappe zu erleichtern. Der Behälter weist im Inneren einen oder mehrere Ringwülste (9) auf, die zur Abgrenzung der Dosiermengen dienen und in gleichen oder unterschiedlichen Abständen voneinander angebracht werden.

Der auf den Behälter aufschiebbare Sprühaufsatz weist neben dem Dichtwulst einen rechtwinkligen Austrittskanal (6) auf. Dami wird der Behälterinhalt beim Aussprühen rechtwinklig in die Wirbelkammer (8) des Sprühkopfes geleitet. Zur Befestigung des Sprühaufsatzes befindet sich im Innern ein erhabener umlaufender Ringwulst (10), der beim Aufschieben des Sprühaufsatzes auf den Behälter in den Ring (11) einrastet. Ausserdem weist der Sprühaufsatz einen Fingerauflagenhalter (17) auf, mit dem das Gerät während der Applikation des Arzneimittels festgehalten werden kann.

Der Behälter kann aus Glas oder Kunststoff hergestellt werden, während der Kolbenstopfen zweckmässigerweise aus Gummi und der Applikator aus Kunststoff gefertigt wird.

Die Montage des Sprühapplikators erfolgt in der Weise, dass der Kolbenstopfen in den Behälter eingeschoben und über das offene Behälterende das Arzneimittel eingefüllt wird. Den bereits vorher zusammengesetzten und sterilisierten Sprühaufsatz schiebt man dann über den noch offenen Behälter und zwar so weit, bis der Ringwulst (10) in den vertieften Ring (11) des Behälters einschnappt. Um den gefüllten Sprühapplikator in Betrieb zu nehmen, wird die Kolbenstange in den Kolbenstopfen eingesetzt, danach die Verschlusskappe abgenommen und dann durch Druck auf die Daumenplatte (18) der Kolbenstopfen in den Behälter hinein geschoben. Beim Auftreffen des Kolbenstopfens auf die im Behälter angebrachten Ringwülste (9) wird der Kolbenstopfen komprimiert und anschliessend mit erhöhtem Kraftaufwand ruckartig in den Dosierbereich bis zum folgenden Ringwulst (9) eingeschoben.

Dieser Vorgang wiederholt sich bei der Entleerung des nächsten Dosierbereichs in gleicher Weise. Bei der praktischen Anwendung, z.B. für den nasalen Bereich, kann der Patient so zuerst die Arzneimittelmenge in die eine Nasenöffnung einsprühen und dann anschliessend diesen Vorgang in der zweiten Nasenöffnung wiederholen.

Gegenüber den bekannten Sprühapplikatoren besitzt der erfindungsgemässe Applikator zahlreiche Vorteile. Der Sprühapplikator wird als Einmaldosierer für mehrere Einzeldosierungen verwendet. Besonders geeignet ist er für sehr kleine Sprühraten (z.B. 0,1 ml/Sprühstoss), wobei Dosierschwankungen aufgrund der exakten Abgrenzung durch die Ringwülste (9) am Behälter verhindert werden.

Ausserdem benötigt dieser Applikator gegenüber bekannten Modellen wesentlich weniger Teile. Insbesondere entfallen Federelemente und komplizierte, bewegliche Dichtungskolben, die nur durch aufwendige Fertigungstechniken herstellbar sind.

Da dieser Sprühapplikator nur zur einmaligen Anwendung bestimmt ist, besteht nicht die Gefahr, dass durch Auskristallisation des Füllgutes und Verschmutzung der Austrittsdüse Verstopfungen eintreten und eine weitere Entnahme verhindert wird. Ausserdem können Füllmengen unter 5 ml für mehrere Dosierungen mit der entsprechenden Anzahl dieser Sprühapplikatoren in einer therapiegerechten Packung zur Anwendung gebracht werden. Falls es erforderlich ist, während der Behandlung unterschiedliche Mengen des Arzneimittels einzusetzen, kann dem dadurch Rechnung getragen werden, dass die durch die Innenringe begrenzten Dosierbereiche in entsprechender, unterschiedlicher Grösser gestaltet werden.

Bei sterilen Füllgütern ist der Sprühapplikator durch die Verschlusskappe gesichert und kann zusätzlich, da er für den einmaligen Gebrauch bestimmt ist, in sogenannte Blister verschweisst werden.

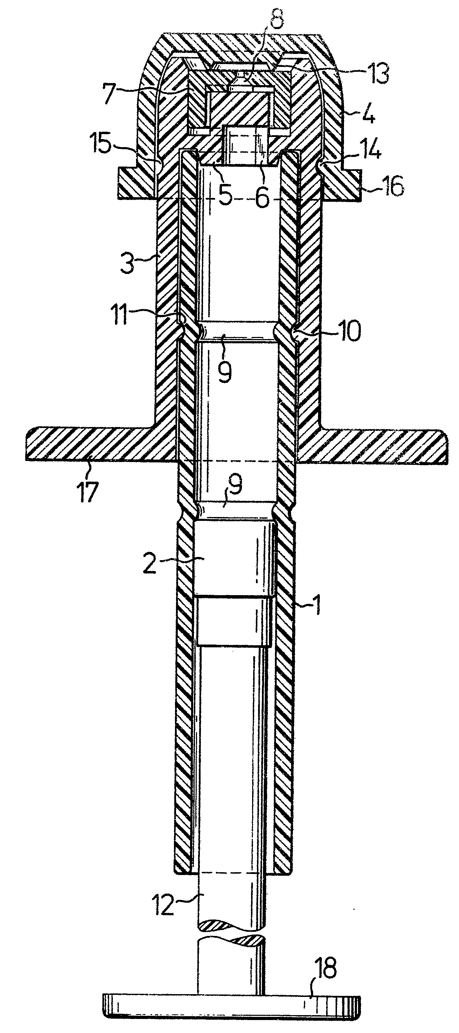

## Patentansprüche

1. Sprühapplikator für flüssige oder pulverförmige Arzneimittel, bei dem der das Arzneimittel enthaltende Behälter (1) an der einen Seite mit einem Kolbenstopfen (2) und auf der anderen Seite mit einem Sprühaufsatz (3) versehen ist, der eine von einer Sprühdüse (7) gebildete Wirbelkammer (8) aufweist, dadurch gekennzeichnet, dass der Behälter (1) aus einem oder mehreren Dosierbereich(en) besteht und der Sprühaufsatz (3) mit einer Verschlusskappe (4) versehen ist, durch einen umlaufenden Dichtwulst (5) gegen

über dem Behälterrand abdichtend wirkt und einen rechtwinkligen Austrittskanal (6) aufweist, durch den der gesamte Arzneimittelstrom in eine radiale Richtung abgelenkt wird und durch den der Arzneimittelstrom rechtwinklig in die Wirbelkammer (8) geleitet und von dort nach aussen gesprüht wird.

2. Sprühapplikator nach Anspruch 1, dadurch gekennzeichnet, dass zur Unterteilung des Behälters in mehrere Dosierbereiche an der Innenseite des Behälters Ringwülste (9) angebracht sind.

3. Sprühapplikator nach Ansprüchen 1 und 2, dadurch gekennzeichnet, dass der Sprühaufsatz mittels eines ringförmigen Wulstes (10), der in einen den Behälter aussen umlaufenden vertieften Ring (11) einschnappt, am Behälter befestigt ist.

4. Sprühapplikator gemäss Ansprüchen 1 bis 3, dadurch gekennzeichnet, dass die Grösse der Dosierbereiche durch unterschiedliche Abstände der Ringwülste (9) festgelegt ist.

## Claims

1. Spray applicator for liquid or pulverulent medicaments, wherein the receptacle (1) containing the medicament is provided with a piston plug (2) at one end, and at the other end with a spray head (3) having a turbulence chamber (8) formed by a spraying nozzle (7), characterized in that the receptacle (1) consists of one or more dosing area(s), and the spray head (3) being provided with a closing cap (4), the spray head (3) acting by means of an annular sealing torus (5) as a seal towards the rim of the receptacle and having a rectangular outlet canal (6), through which the complete stream of the medicament is directed into a radial direction and through which the stream of the medicament is led at a right angle into the turbulence chamber (8) and from there sprayed to the outside.

2. Spray applicator according to Claim 1, characterized in that annular tori (9) are arranged at the inner face of the receptacle in order to divide it into several dosing areas.

3. Spray applicator according to Claims 1 and 2, characterized in that the spray head is fastened to the receptacle by means of an annular torus (10) catching in an annular groove (11) at the outer face of the receptacle.

4. Spray applicator according to Claims 1 to 3, characterized in that the size of the dosing areas is determined by differing distances of the annular tori (9).

## Revendications

1. Pulvérisateur pour médicaments liquides ou en forme de poudre, dans lequel le récipient (1) contenant le médicament est muni d'un côté d'un bouchon-piston (2) et, de l'autre côté, d'un embout de pulvérisation (3), qui présente une chambre de turbulence (8) formée par un gicleur de pulvérisation (7), caractérisé en ce que le récipient (1) est constitué par une ou plusieurs région(s) de do

sage et que l'embout de pulvérisation est muni d'un capuchon de fermeture (4), et forme un joint d'étanchéité par rapport au bord du récipient au moyen d'un bourrelet d'étanchéité (5), cet embout présente un canal de sortie (6) à angle droit par lequel tout le courant de médicament est dévié dans une direction radiale et par lequel le courant de médicament est conduit à angle droit dans la chambre de turbulence (8) et, de là, pulvérisé vers l'extérieur.

2. Pulvérisateur suivant la revendication 1, caractérisé en ce que, pour diviser le récipient en plusieurs régions de dosage, des bourrelets annulaires (9) sont prévus sur la face interne du récipient.

3. Pulvérisateur suivant les revendications 1 et 2, caractérisé en ce que l'embout de pulvérisation est fixé au récipient au moyen d'un bourrelet (10) de forme annulaire qui s'encliquette dans un anneau creux (11) qui entoure extérieurement le récipient.

4. Pulvérisateur suivant les revendications 1 à 3, caractérisé en ce que la grandeur des régions de dosage est fixée par différentes distances des bourrelets annulaires (9).

0 021 123